Europäisches Patentamt

European Patent Office

Office européen des brevets

(11) Veröffentlichungsnummer: **0 252 368**
**A2**

# (12) EUROPÄISCHE PATENTANMELDUNG

(21) Anmeldenummer: **87109063.5**

(51) Int. Cl.⁴: **C12Q 1/26 , C12N 9/00**

(22) Anmeldetag: **24.06.87**

Der (Die) Mikroorganismus (Mikroorganismen) ist (sind) bei der Deutschen Sammlung von Mikroorganismen unter der (den) Nummer(n) DSM 10, DSM 322, DSM 609 hinterlegt worden.

(30) Priorität: 05.07.86 DE 3622620
04.02.87 DE 3703315

(43) Veröffentlichungstag der Anmeldung:
13.01.88 Patentblatt 88/02

(84) Benannte Vertragsstaaten:
BE CH DE FR GB IT LI NL SE

(71) Anmelder: **Merck Patent Gesellschaft mit beschränkter Haftung**
**Frankfurter Strasse 250**
**D-6100 Darmstadt(DE)**

(72) Erfinder: **Scholz, Bernard, Dr.**
**Mozartstrasse 10**
**D-6087 Büttelborn(DE)**
Erfinder: **Ebeling, Wolfgang, Dr.**
**Am Hintergraben 9**
**D-6101 Bickenbach(DE)**
Erfinder: **Wahl, Hans Peter, Dr.**
**Lindenweg 22**
**D-6107 Reinheim(DE)**
Erfinder: **Vormbrock, Rolf, Dr.**
**Greinstrasse 10c**
**D-6100 Darmstadt(DE)**
Erfinder: **Helger, Roland, Dr.**
**Ludwigshöhstrasse 85**
**D-6100 Darmstadt(DE)**
Erfinder: **Metz, Harald, Prof. Dr.**
**Am Landbach 8**
**D-6101 Bickenbach(DE)**
Erfinder: **Brümmer, Wolfgang, Dr.**
**Am Grenzweg 9**
**D-6146 Alsbach(DE)**
Erfinder: **Linxweiler, Winfried, Dr.**
**Dieselstrasse 2**
**D-6101 Messel(DE)**

(54) **Verfahren und Reagenz zur Bestimmung von Creatinin sowie dazu geeignetes Enzym.**

(57) Die Erfindung betrifft ein Verfahren und ein Reagenz zur enzymatischen Bestimmung von Creatinin sowie ein Enzym mit Creatinin:NAD(NADP) oxidoreductase-Aktivität und dessen Herstellung aus Mikroorganismen der Gattung Bacillus oder aus Glucosedehydrogenase.

Das Enzym oxidiert Creatinin und überträgt dabei zwei Wasserstoffatome auf NAD (NADP) unter Bildung von NADH (NADPH), das als Meßgröße dient.

EP 0 252 368 A2

## Verfahren und Reagenz zur Bestimmung von Creatinin sowie dazu geeignetes Enzym

Die Erfindung betrifft ein Verfahren und ein Reagenz zur enzymatischen Bestimmung von Creatinin sowie ein Enzym mit Creatinin: NAD(NADP) oxidoreductase-Aktivität und dessen Herstellung.

In der klinischen Chemie kommt der Bestimmung von Creatinin im Serum und Harn eine zentrale Bedeutung bei der Diagnose der Ausscheidungsfähigkeit der Niere, der sogenannten Creatinin-Clearance, zu. Darüber hinaus wird die Menge von mit dem Harn ausgeschiedenen Substanzen (Metaboliten, Serumproteine und Enzyme) üblicherweise nicht auf die wechselnden Harnvolumina, sondern auf die konstant ausgeschiedene Menge an Creatinin bezogen. Die Methode nach Jaffe, bei der der Probe Pikrinsäure zugesetzt wird und nach Alkalisieren der rote Farbstoff gemessen wird, ist heute noch die am häufigsten angewendete Methode zur Bestimmung von Creatinin. Zahlreiche Störungen des Nachweissystems, vor allem durch sogenannte Creatininchromogene, machen es erforderlich, aufwendige Schritte wie Enteiweißung, Adsorption an Fullererde oder die unterschiedliche Bildungskinetik anzuwenden.

In der DE-OS 21 22 255 ist ein enzymatisches Verfahren zur spezifischen Bestimmung von Creatinin unter Verwendung des Enzyms Creatininamidohydrolyase beschrieben. Creatinin wird dabei zu Creatin umgesetzt und das gebildete Creatin wird nach einer Reihe von Hilfsreaktionen durch den Verbrauch von NADH quantitativ bestimmt. Der Vorteil des Verfahrens besteht in der hohen Spezifität. Diese muß jedoch durch eine umständliche Versuchsdurchführung erkauft werden, die neben der eigentlichen Bestimmung des Analyten auch Bestimmungen des Probenleerwertes und des Reagenzienleerwertes erfordert. Dazu kommt die Prüfung des Überverbrauchs an NADH und gegebenenfalls die Wiederholung der Analyse nach Verdünnung. Die dem Fachmann bekannte geringe Stabilität des dabei erforderlichen Hilfsenzyms Creatinkinase schränkt die Wirtschaftlichkeit dieser Methode, insbesondere bei kleinen Probenzahlen, erheblich ein.

In J.Biol.Chem. 121, 429 (1937) wurde die durch Creatininase katalysierte Umsetzung des Creatinins zu Creatin und dessen Weiterreaktion zu Sarcosin und Harnstoff beschrieben. Der entstandene Harnstoff läßt sich mittels Urease zu Ammoniak umsetzen, das über die Farbänderung eines pH-Indikators oder elektrochemisch bestimmt wird. Harnstoff kommt jedoch als ausscheidungspflichtiges Entgiftungsprodukt des Ammoniaks im Serum und im Harn vor; im Harn sogar in wesentlich höherer Konzentration als Creatinin. Es muß also in meßtechnisch ungünstiger Weise auf ein großes Signal aus endogenem Harnstoff ein wesentlich kleineres Signal aus Creatinin aufgesetzt werden.

Bei einer Reihe weiterer Verfahren wird das Sarcosin als Schlüsselsubstanz verwendet (DE-OS 32 48 145). Sarcosin wird dabei mittels Sarcosinoxidase unter anderem zu Wasserstoffperoxid umgesetzt, das in bekannter Weise zur Farbstoffbildung verwendet wird. Um den Einfluß endogenen Creatins zu eliminieren, muß ein Probenleerwert gemessen oder das Creatin vorher entfernt werden. Das System unterliegt den bekannten Störungen des Peroxidasesystems; es muß, zumindest um die Ascorbatstörung zu beseitigen, eine große Menge an Ascorbatoxidase zugesetzt werden. Die Verwendung von vier bis sechs Enzymen und die Bestimmung von Probe und Probenleerwert verteuert die Einzelbestimmung.

Es besteht daher immer noch ein dringendes Bedürfnis nach einem spezifischen Test zur Bestimmung von Creatinin, der sowohl einfach und damit gut handhabbar als auch störungsfrei ist. Der Erfindung liegt die Aufgabe zugrunde, der Fachwelt einen solchen Test zur Verfügung zu stellen.

Gegenstand der Erfindung ist ein Verfahren zur enzymatischen Bestimmung von Creatinin, das dadurch gekennzeichnet ist, daß eine wäßrige, Creatinin enthaltende Lösung mit einem Enzym mit Creatinin:NAD-(NADP) oxidoreductase-Aktivität zusammen mit NAD oder NADP inkubiert und das gebildete NADH bzw. NADPH bestimmt wird.

Ein weiterer Gegenstand der Erfindung ist ein Reagenz zur enzymatischen Bestimmung von Creatinin, das ein Enzym mit Creatinin:NAD(NADP) oxidoreductase-Aktivität und NAD oder NADP enthält.

Die Erfindung betrifft ferner ein Enzym mit Creatinin: NAD(NADP) oxidoreductase-Aktivität sowie Verfahren zu dessen Herstellung. Die Verfahren sind dadurch gekennzeichnet, daß man Mikroorganismen der Gattung Bacillus oder mit den entsprechenden Bacillusgenen transformierte Escherichia coli-Stämme in einem üblichen Medium züchtet, aufschließt und das im Rohextrakt enthaltene Enzym nach chromatographischer und elektrophoretischer Aufreinigung mit die Polarität der wäßrigen Lösung herabsetzenden Substanzen versetzt oder mit Glucose oder Glucosederivaten kovalent verknüpft.

Das erfindungsgemäße Verfahren zur Bestimmung von Creatinin wird mit dem Enzym mit Creatinin:NAD(NADP) oxidoreductase-Aktivität durchgeführt, das überraschenderweise ausschließlich Creatinin oxidiert und dabei zwei Wasserstoffatome auf NAD oder NADP unter Bildung von NADH bzw. NADPH überträgt. Eine Reaktion mit Creatin findet nicht statt, ebensowenig mit anderen linearen und zyklischen, substituierten und nichtsubstituierten Guanidinoverbindungen, zu denen auch Sarcosin gehört. Das gebildete NADH (NADPH) wird in bekannter Weise spektrophotometrisch, fluoreszenzspektroskopisch oder nach Umsetzung mit Tetrazoliumsalzen über die Menge des gebildeten Formazans bestimmt.

Die erfindungsgemäße Creatininbestimmung wird bei einem pH-Wert zwischen 3,5 und 9,5 durchgeführt, vorzugsweise zwischen 5,0 und 7,5. Zur Einstellung des pH-Wertes können alle Puffer verwendet werden, die das Indikatorsystem nicht stören. Geeignete Puffer sind z.B. N-(2-Acetamido)-2-aminoethansulfonsäure (ACES), N-(2-Acetamido)iminodiessigsäure (ADA), Ammoniumcarbonat, Ammoniumformiat, Ammoniumacetat, Citrat, 2-Amino-2-methyl-1,3-propandiol (AMP), Borat, N,N-Bis(2-hydroxyethyl)glycin (BICIN), Collidin, Ethylendiamin, N-(2-Hydroxyethyl) piperazin-N'-3-propansulfonsäure (EPPS), Glycin, Glycylglycin, N-(2-Hydroxyethyl)piperazin-N'-2-ethansulfonsäure (HEPES), Imidazol und substituierte Imidazole, Morpholinoethansulfonsäure (MES), Morpholinopropansulfonsäure (MOPS), Piperazin-N,N'-bis(2-ethansulfonsäure) (PIPES), Phosphat, N-Tris(hydroxymethyl)methyl-3-aminopropansulfonsäure (TAPS), N-Tris(hydroxymethyl)-methyl-2-aminoethansulfonsäure (TES), Tris(hydroxymethyl)methylglcyin (Tricin), Triethanolamin und/oder Tris(hydroxymethyl)aminomethan (Tris) sowie andere, in der Biochemie geläufige Puffer. Bevorzugte Puffer sind z.B. Phosphat, Glycin, Tris oder TES. Der Glycin/Tris-Puffer (100 mM/l, pH 9,0) bewirkt gegenüber dem Phosphatpuffer eine um den Faktor 3,4 gesteigerte Aktivität. Die Konzentrationen der verwendeten Puffer sollten im Bereich zwischen 10 und 500 mmol, vorzugsweise zwischen 30 und 250 mmol liegen. Das pH-Optimum liegt bei Verwendung des Phosphatpuffers bei pH 8,0, bei Verwendung von Glycin/NaOH-Puffer oder Glycin/Tris-Puffer bei pH 9,0, bei Verwendung von Citratpuffer bei pH 5,0 und bei TES-Puffer bei pH 5,4.

Die Bestimmung wird vorzugsweise in Gegenwart von Substanzen durchgeführt, die die Polarität wäßriger Lösungen herabsetzen und damit die Aktivität gegeniber Creatinin steigern. Dieser Effekt kann durch Zusatz von wasserlöslichen anorganischen oder organischen Verbindungen erreicht werden, die dissoziierbar oder nichtdisoziierbar sind und eine starke Hydrathülle aufbauen. Beispiele für derartige Verbindungen sind Ammoniumsulfat, Alkohole, darunter auch Polyole, Dimethylsulfoxid, Aceton, Dimethylformamid, höherkettige aliphatische, cycloaliphatische, aromatische, gemischt langkettigcyclische und gemischt araliphatische Ketone, Aldehyde und Alkohole, die pro Moleküleinheit 1-6 Hydroxylgruppen tragen sowie deren intra-oder intermolekularen Ketale bzw. Acetale.

Bevorzugt werden Polyole wie Mono-, Di-, Oligo-oder Polysaccharide eingesetzt, insbesondere Saccharose, Galactose, Maltose und/oder Mannose. Die Konzentration dieser Aktivatoren sollte zwischen 0,05 und 20 Gew.-% liegen. Bevorzugt ist z.B. eine Konzentration von etwa 5 % für Galactose, 7 % für Saccharose und 15 % für Mannose. Die Menge des Aktivators richtet sich auch nach der Reinheit des Enzyms. Vorzugsweise wird das Enzym zusammen mit dem Aktivator gelöst und frühestens zehn Minuten nach Durchmischung zur Bestimmung eingesetzt.

Eine weitere Möglichkeit der Aktivierung des Enzyms mit Creatinin: NAD(NADP) oxidoreductase-Aktivität besteht im Einbau in natürliche oder synthetische Membranen (Liposomen, Micellen), wie es z.B. in Liposomes 41, 1985, für andere membrangebundene Enzyme beschrieben ist. So hat z.B. der Einbau in Octylglycosid eine Aktivitätssteigerung um den Faktor 3,1 zur Folge.

Es ist auch vorteilhaft, eine eventuell noch vorhandene Glucose-umsetzende Aktivität durch Zusatz von Inhibitoren wie Galakturonsäure, Streptomycinsulfat, Gluconsäurelacton, Octylglycosid, vorzugsweise Galakturonsäure,zu inhibieren.

Die NADH-bzw. NADPH-Bildung der erfindungsgemäßen Reaktion verläuft quantitativ und ermöglicht dadurch eine absolut spezifische und quantitative Bestimmung des Creatinins. Die Auswertung erfolgt über die Bestimmung des gebildeten NADH bzw. NADPH in an sich bekannter Weise. Das gebildete NADH bzw. NADPH kann über die UV-Absorption bei einer Meßwellenlänge von 340 nm oder 365 nm, über das Fluoreszenzsignal oder nach katalytischer Umsetzung von NADH (NADPH) mit Tetrazoliumsalzen in Gegenwart des Enzyms Diaphorase oder des Elektronenüberträgers Phenazinmethosulfat spektrophotometrisch bestimmt werden. Das Meßverfahren nach der Erfindung ist unempfindlich gegenüber Störungen durch Creatinin, Sarcosin, Creatinchromogene und, soweit die NADH-Bildung direkt gemessen wird, gegenüber Redoxverbindungen, die bekannterweise Peroxidasesysteme stören. Wird die Reaktion über die Bildung von Formazanfarbstoffen verfolgt, können die letztgenannten Störungen ebenfalls durch die Wahl eines geeigneten Tetrazoliumsalzes eliminiert werden.

Das erfindungsgemäße Reagenz eignet sich auch in Form von damit imprägnierten saugfähigen Trägermaterialien oder eingearbeitet in Folien als Indikator zum Nachweis von Creatinin. Als Trägermaterialien eignen sich die für analytische Nachweisreagenzien üblichen Träger wie Papier, Cellulose, Faservlies, poröse Kunststoffmembranen oder ähnliche Materialien.Die Herstellung erfolgt durch Eintauchen oder Besprühen mit dem erfindungsgemäßen Reagenz.

Die Herstellung des Enzyms mit Creatinin:NAD(NADP) oxidoreductase-Aktivität erfolgt durch Züchtung geeigneter Mikroorganismen der Gattung Bacillus und Gewinnung des Enzyms aus der Biomasse, aus dem Lysat oder aus der Kulturbrühe. Als besonders geeignet erwiesen sich Mikroorganismen der Gattung Bacillus, die Glucosedehydrogenase bilden können, z.B. Bacillus megaterium, Bacillus cereus, Bacillus subtilis oder auch mit den entsprechenden Bacillusgenen transformierte Escherichia coli-Stämme. Die Mikroorganismen der Gattung Bacillus sind häufig vorkommende Organismen und von allen Stammsammlungen erhältlich.

Geeignet sind übliche Nährmedien, die das Wachstum von Bacillus fördern. Ein bevorzugtes Nährmedium, das sogenannte L-Broth-Medium, enthält 1 % Bacto Trypton, 0,5 % Hefeextrakt, 0,5 % Kochsalz und destilliertes Wasser. Der pH-Wert wird auf 7,2 eingestellt. Bei der Aufzucht der Bacilli hat sich eine Hefeextrakt-Pepton-haltige Nährlösung bewährt, die 0,5 % Hefeextrakt, 0,1 % Pepton aus Casein, 0,2 % Cornsteep, 0,6 % Glucose und 33 mM Phosphatpuffer, pH 7,0, enthält. Neben ähnlichen komplexen Nährlösungen sind auch synthetische Nährmedien geeignet, wie z.B. 0,2 % Ammoniumphosphat, 0,4 % Glucose, 0,4 % Natriumglutaminat, Mineralsalze und Spurenelemente, eingestellt auf einen pH-Wert von 6,8.

Zur Isolierung des Enzyms mit Creatinin: NAD(NADP) oxidoreductase-Aktivität schließt man den in diesen Medien gezüchteten Mikroorganismus auf und trennt vom Unlöslichen ab. Für den Aufschluß eignen sich die gebräuchlichen Methoden wie Hochdruckdispersion, Ultraschall, Desintegrationsmühle und/oder Lysozymzusatz. Bevorzugt ist der Zusatz von Lysozym alleine oder eine Mischung von Lysozym und Detergentien wie Octylglycosid oder Polyethylenglycolalkylphenylether. Nach Zentrifugation bei 100 000 × g wird dem klaren Überstand Ammoniumsulfat bis zur Endkonzentration von 55 % zugesetzt. Das Präzipitat wird entfernt und verworfen, die überstehende Lösung mit Ammoniumsulfat bis zur Konzentration von 80 % aufgestockt. Das innerhalb von zwei Stunden gebildete Präzipitat wird aufgelöst und über Fractogel HW 55 (Elutionsmittel: Phosphatpuffer 100 mM, pH 7,5) von nicht aktivem Fremdprotein abgetrennt. Der Aufreinigungsfaktor für beide Operationen beträgt, abhängig vom Züchtungserfolg, etwa 50. Die weitere Aufreinigung erfolgt an einem hydrophoben Trägermaterial in 50 mM Phosphatpuffer, pH 8,5. Einen ähnlichen Reinigungseffekt erreicht man an Farbstoffgelen, z.B. an Procion Rot HE 38.

Schließlich wird die so erhaltene Glucosedehydrogenase in der free-flow-Elektrophorese bei pH 9,0 (Glycin/Tris-Puffer 50 mM, pH 3,0, Proteinkonzentration 50 μg/ml max. nach einstündiger Vorinkubation) von den verbleibenden Fremdproteinen abgetrennt.

Die für die Creatininbestimmung erforderliche Aktivität wird entweder durch den Zusatz von die Polarität herabsetzenden Substanzen gegebenenfalls in Gegenwart von Inhibitoren oder vorzugsweise durch kovalente Verknüpfung der Glucosedehydrogenase mit Glucose oder Glucosederivaten erhalten. Aufgrund der sehr hohen Michaelis-Konstante der Glucosedehydrogenase ($4,75 . 10^{-2}$ M Glucose) war nicht zu erwarten, daß die Aktivität der Glucosedehydrogenase gegenüber Glucose durch eine chemische Blockierung der Glucosebindungsstellen mit kovalent gebundener Glucose, Glucosederivaten oder anderen Zuckern ausgeschaltet werden kann. Umso überraschender war es, daß durch chemische Modifizierung die Glucosedehydrogenase derart verändert werden konnte, daß eine neue Substratspezifität gegenüber einem strukturell völlig verschiedenen Substrat resultiert. Diese katalytische Wirkung ist keine Nebenaktivität, sondern die einzige Aktivität des erhaltenen Enzyms.

Die Herstellung des Enzyms mit Creatinin:NAD(NADP) oxidoreductase-Aktivität erfolgt entweder aus den oben beschriebenen Glucosedehydrogenase bildenden Bacillusarten oder auch aus kommerziell erhältlicher Glucosedehydrogenase. Geeignete Reaktionspartner für die Blockierung der Glucosedehydrogenase sind Glucose, Mannose, Xylose, D-Arabinose, L-Rhamnose, D-Mannit, myo-Inosit, D-Glucosamin, N-Acetylglucosamin, vorzugsweise Derivate dieser Zucker, wie in 1-Stellung halogenierte Glucose und deren Derivate, insbesondere 1-Brom-2,3,4,6-tetraacetylglucose und in situ diazotiertes Glucosamin.

Die Bindung an die Aminogruppen des Enzymes erfolgt vorzugsweise über die freie Carbonylgruppe der nichtzyklischen Form des Zuckers oder über die Addition der carbokationischen Form der Glucose, die sich nach der Diazotierung von Glucosamin oder deren Derivate mit Nitrit in saurer Lösung spontan bildet. Eine besonders bevorzugte Kupplung nach der Erfindung ist die nucleophile Substitutionsreaktion an in 1-Position halogenierter Glucose und deren Derivate in Gegenwart geeigneter Lewis-Basen, wie ammoniakalische Silbersalzlösung, z.B. ammoniakalisches Silbercarbonat oder Silbernitrat.

4

Zur Herstellung des Enzyms mit Creatinin:NAD(NADP) oxidoreductase-Aktivität wird Glucosedehydrogenase in einem neutralen Puffer gelöst, mit einem Glucosederivat versetzt und anschließend eine Lewis-Base zugegeben. Je nach Dauer der Reaktionszeit bei Raumtemperatur nimmt die Aktivität der Glucosedehydrogenase ab, bis sie schließlich völlig verschwunden ist, während die Aktivität der Creatinin:NAD-(NADP) oxidoreductase unverändert erhalten bleibt oder sich noch erhöht. Das Reaktions gemisch wird durch geeignete Methoden, wie Dialyse, Gelfiltration, Präzipitation der Silbersalze oder Ionenaustauscherchromatographie gereinigt und kann direkt zur Creatininbestimmung eingesetzt werden. Bei dem auf diese Weise glykosylierten Enzym kann auf die im oben beschriebenen alternativen Herstellungsverfahren erforderliche Aktivierung durch Mannose, Galaktose, Saccharose und/oder Maltose verzichtet werden.

Das erfindungsgemäße Enzym mit Creatinin:NAD(NADP) oxidoreductase-Aktivität läßt sich ohne Aktivitätsverlust bei -20 °C oder als Ammoniumsulfatsuspension bei +4 °C mindestens drei Monate lang aufbewahren. In 3M NaCl-Lösung ist das Enzym bei Kühlschranktemperatur mehr als 6 Monate haltbar. In 3M NaCl-Lösung erleidet es bei 60 °C innerhalb einer halben Stunde keinen Aktivitätsverlust. Der isoelektrische Punkt liegt bei pH 7,5 und das Molekulargewicht beträgt etwa 120 000 Dalton. In 50 mM Glycin/Tris-Puffer vom pH-Wert 9,0 (weniger als 50 μg Protein pro ml) wird das Enzym inaktiv. Mindestens 90 % der ursprünglichen Aktivität lassen sich nach Erhöhung der Ionenstärke oder nach Änderung des pH-Wertes auf 7,5 zurückgewinnen. Das Enzym adsorbiert an Sephadex-Gel und an Polyacrylnitril.

Für das erfindungsgemäße Verfahren zur Bestimmung von Creatinin kann der Zellüberstand nach Lyse, das aufgereinigte Enzym oder das aufgereinigte Reaktionsprodukt der Kupplungsreaktion eingesetzt werden. Vorzugsweise setzt man das aufgereinigte Reaktionsprodukt der Kupplungsreaktion ein.

## Beispiel 1

Reaktionslösung:
   50 mmol/l NAD
   5 % Galaktose (w/v)
   300 mmol/l Galakturonsäure
   5 U Creatinin:NAD(NADH) oxidoreductase-Aktivität in 50 mM Kaliumnatriumphosphatpuffer, pH 7,5

Die Reaktionslösung wird auf 25 °C temperiert. Zu 1 ml Reaktionslösung werden 0,1 ml einer Creatinin enthaltenden Probe zugesetzt und bei 25 °C eine halbe Stunde lang inkubiert. Die Extinktionsdifferenz $\Delta E$ zwischen der Extinktion $E_1$ unmittelbar nach Durchmischen und der Extinktion $E_2$, die sich nach einer halben Stunde ergibt, wird ermittelt und daraus die Creatininkonzentration wie folgt errechnet:

$$c = 1770 \; \frac{\Delta E}{d} \; \mu mol/l \; oder$$

$$c = 20.04 \; \frac{\Delta E}{d} \; mg/dl$$

Die folgende Tabelle zeigt die Linearität zwischen der Konzentration c des Analyten in der zugesetzten Probe und der Extinktionszunahme E.

| c (mg/dl) | (µmol/l) | E |
|---|---|---|
| 0.3 | 26.5 | 0.020 |
| 0.6 | 53.1 | 0.032 |
| 0.9 | 80.0 | 0.047 |
| 1.2 | 106.0 | 0.058 |
| 2.0 | 177.0 | 0.101 |
| 3.0 | 265.4 | 0.147 |
| 4.0 | 353.9 | 0.201 |
| 5.0 | 442.5 | 0.249 |
| 7.5 | 663.7 | 0.378 |
| 10.0 | 884.9 | 0.501 |
| 15.0 | 1327.3 | 0.746 |
| 20.0 | 1769.9 | 1.000 |
| 25.0 | 2212.4 | 1.240 |
| 30.0 | 2654.9 | 1.480 |
| 40.0 | 3539.8 | 1.998 |

Beispiel 2

Zu 2 ml Reaktionslösung bestehend aus

10 mmol/l NAD
7,5 % Saccharose (w/v)
10 U Creatinin:NAD(NADH) oxidoreductase-Aktivität
in 20 mM Tris/HCl-Puffer, pH 8,5

werden 50 µl Probenlösung zugesetzt. Die Zunahme des Fluoreszenssignals $\Delta I/\Delta t$ wird mit einem Spektralfluorimeter mit Quarz-Durchflußküvette gemessen.
Geräteeinstellung:
Anregungswellenlänge  380 nm
Emissionswellenlänge  480 nm
Anregungsspalt  5 mm
Emissionsspalt  10 mm
Verstärkereinstellung  × 10
Eine Zunahme der Fluoreszenzintensität von 4 Einheiten/min entspricht einer Creatininkonzentration von 1.27 µmol/l in der zugesetzten Probe.

Beispiel 3

Reaktionslösung:

20 mmol/l NAD
2 % Mannose
1 mmol/l 3-(4-Jodphenyl)-2(4-nitrophenyl,-5-phenyl-2H-tetrazoliumchlorid (INT)
20 U Diaphorase in
100 mM Tris/NaOH-Puffer, pH 9,0

6

An Stelle von INT sind auch andere Tetrazoliumsalze wie 3-(4,5-Dimethylthiazol-2-yl)-2,5-diphenyl-2H-tetrazoliumbromid, Nitroblau-Tetrazoliumchlorid, Tetranitroblau-Tetrazoliumchlorid, Tetrazolblau und 2,3,5-Triphenyltetrazoliumchlorid verwendbar. 1 ml Reaktionslösung wird mit 0,1 ml Probelösung versetzt und 5 Min. inkubiert.

Durch Zusatz von 5 U Creatinin:NAD(NADP) oxidoreductase-Aktivität wird die Reaktion gestartet und die Extinktionserhöhung bei $\lambda$ = 640 nm nach 30 Min. gemessen. Eine Extinktionserhöhung von 0.1 E entspricht einer Creatininkonzentration von 76 $\mu$mol/1 in der zugesetzten Probe.

Beispiel 4

Teststreifen

Eine Lösung von
10 mmol/l NAD
2 % Mannose
3 mmol/l INT
200 U/l Diaphorase
1,5 mol/l Kochsalz
2,5 g/l Polyvinylpyrrolidon,
gelöst in 100 mmol/l Tris-Puffer,
pH 7,2,

wird mit 257 U/Liter Creatinin:NAD(NADP) oxidoreductase-Aktivität versetzt. Nach Lösen wird durch ein 0,22 $\mu$ Membranfilter filtriert und ein Filtrierpapier (1450 CV der Fa. Schleicher und Schull) mit dieser Lösung getränkt und getrocknet. Das getrocknete Papier wird in 6 $\times$ 6 mm große Quadrate geschnitten und auf 6 mm breite Polystyrolstreifen aufgebracht.

Bei Benetzung mit creatininhaltigen Flüssigkeiten verfärbt sich innerhalb einer Minute die Reaktionszone blau. Die Farbtiefe ist vom Creatiningehalt abhängig.

Beipiel 5

Herstellung des Enzyms

Bacillus megaterium DSM 322 wird in einer Nährlösung aus

0,5 % Hefeextrakt
0,1 % Pepton aus Casein
0,2 % Cornsteep
0,6 % Glucose
33 mM Phosphatpuffer, pH 7.0

im 11-Glasfermenter zwischen 28 und 42 °C kultiviert. (Rührgeschwindigkeit: 400 U/min, Belüftung: 400 l/h). Die Zellen werden in einer Zentrifuge bei 30.000 $\times$ g abzentrifugiert, das Zentrifugat in 0,1 M Kaliumphosphatpuffer, pH 6,5, resuspendiert und nach Zusatz von EDTA in einer Konzentration von 20 mM sowie von Lysozym (5 g/l Suspension) und Octylglykosid (26 mM) so lange lysiert, bis das Maximum an Enzymaktivität erreicht wird. Die Zellfragmente werden bei 120.000 $\times$ g innerhalb einer Stunde abzentrifugiert, der klare Überstand auf + 4 °C gebracht und mit festem Ammoniumsulfat bis zur Konzentration von 55 % versetzt. Nach einer Stunde wird zentrifu@iert (80.000 $\times$ g), das Präzipitat verworfen und der Überstand mit festem Ammoniumsulfat auf 80 % aufgestockt und über Nacht bei +4 °C gerührt. Das gebildete Präzipitat wird bei 80.000 $\times$ g innerhalb einer Stunde in der Ultrazentrifuge isoliert, in 100 mM Phosphatpuffer, pH 7,5, gelöst und über eine Gelfiltrationssäule (Fractogel TSK HW 55 F) chromatographiert. Die enzymhaltigen Faktionen werden vereinigt, über eine Dialysemembran (Ausschlußvolumen 10.000 Dalton) aufkonzentriert und über eine Trisacryl 2000-Säule mit anschließender Elektrophorese oder durch Ionenaustauscher-Chromatographie (Mono-Q von Pharmacia), aufgearbeitet. Während des Pufferwechsels auf 100 mM Tris-Puffer, pH 7,5, in der Mikrodialysezelle fällt weiteres, inaktives Protein aus, das verworfen wird. Der Überstand wird im Kochsalzgradienten von der Ionenaustauschersäule eluiert. Die Fraktionen zwischen 280 und 320 mM Kochsalz enthalten das elektrophoretisch reine Enzym. Die spezifische Aktivität liegt bei etwa 15 U/mg. Ähnliche Ergebnisse werden mit den Bacillus megaterium-Stämmen DSM 333, DSM 337, DSM 339 und DSM 344 erhalten.

100 mg der so hergestellten Glucosedehydrogenase werden in 10 ml 0,2 M Kaliumphosphatpuffer, pH 7,0, gelöst und über Nachtgegen 1 l Puffer dialysiert. Die Lösung wird mit dem gleichen Puffer auf einen Proteingehalt von 1,0 mg/ml (Coomassie-Test, Eichung mit BSA) eingestellt. In 3 ml dieser Lösung werden 50 mg 1-Brom-2,3,4,6-tetracetylglucose gelöst und 30 Min. inkubiert.

Unter Rühren wird der abgedunkelten Lösung 1 ml einer Lösung zugesetzt, die 100 mg Silbercarbonat in 2 ml 2,5 %iger wässriger Ammoniaklösung enthält, und nach bestimmten Reaktionszeiten analysiert. Dazu werden Aliquote entnommen und über eine Dialysemembran mit Ausschlußvolumen 10 k Dalton mit dem 5fachen Volumen 0,2 M Kaliumphosphatpuffer, pH 7,0, kontinuierlich dialysiert. Die Proteinlösungen werden auf gleichen Proteingehalt eingestellt und daraus die Glucosedehydrogenase-und Creatinin: NAD-(NADP) oxidoreductase-Aktivitäten bestimmt. Die folgende Tabelle zeigt den zeitlichen Verlauf der Aktivitätsveränderungen.

| Inkubationszeit | Aktivität [%] | |
| --- | --- | --- |
| | Glucose-dehydrogenase | Creatinin:NAD(NADP)-oxidoreductase |
| 0 min | 100 % | 100 % |
| 15 min | 57 % | 98 % |
| 2 h | 10,7 % | 113 % |
| 1 d | 3,8 % | 160 % |
| 2 d | 2,3 % | 210 % |
| 3 d | 1,2 % | 260 % |
| 6 d | 0,6 % | 295 % |
| 11 d | 0,4 % | 330 % |

Die Ergebnisse zeigen, daß nach 11 Tagen die Glucosedehydrogenase-Aktivität fast auf Null abgesunken ist und die Aktivität der Creatinin:NAD(NADP) oxidoreductase um den Faktor 3,3 gestiegen ist.

Die Bestimmung der Enzymaktivitäten wurde wie folgt durchgeführt:

a) Glucosedehydrogenase

2,0 ml 0,12 M Phosphatpuffer, pH 7,6,
1,0 ml D-Glucose (0,1 g/ml) im gleichen Phosphatpuffer,
0,05 ml Enzympräparation und
0,05 ml NAD (80 mg/ml) in destilliertem Wasser
werden bei 25 °C inkubiert und die Änderung der Extinktion bei der Wellenlänge 340 nm oder 366 nm gemessen.

$E_{340} = 6,3$ cm$^2$/$\mu$mol
$E_{366} = 3,4$ cm$^2$/$\mu$mol

Eine Einheit reduziert 1 $\mu$mol NAD pro Minute unter Testbedingungen.

b) Creatinin:NAD(NADP) oxidoreductase

2,0 ml 1 M Citratpuffer, pH 5,0,
0,5 ml Creatinin (51 mg/ml in 1 M Citratbuffer pH 5,0),
0,5 ml NAD (115 mg/ml in 1 M Citratbuffer pH 5,0),
0,1 ml Enzympräparation mit 1 % Mannose

werden bei 25 °C inkubiert und die Änderung der Extinktion bei der Wellenlänge 366 nm gemessen.

Eine Einheit reduziert 1 μmol NAD pro Minute unter Testbedingungen.

## Ansprüche

1. Verfahren zur enzymatischen Bestimmung von Creatinin, dadurch gekennzeichnet, daß eine wäßrige, Creatinin enthaltende Lösung mit einem Enzym mit Creatinin: NAD(NADP) oxidoreductase-Aktivität zusammen mit NAD oder NADP inkubiert und das gebildete NADH bzw. NADPH bestimmt wird.

2. Reagenz zur enzymatischen Bestimmung von Creatinin, enthaltend ein Enzym mit Creatinin:NAD-(NADP) oxidoreductase-Aktivität und NAD oder NADP.

3. Reagenz nach Anspruch 2, dadurch gekennzeichnet, daß es zusätzlich die Polarität der wäßrigen Lösung herabsetzende Substanzen enthält.

4. Reagenz nach den Ansprüchen 2 und 3, dadurch gekennzeichnet, daß es das Enzym mit Creatinin:NAD(NADP) oxidoreductase-Aktivität in Membranen eingebaut enthält.

5. Verfahren zur Herstellung eines Enzyms mit Creatinin:NAD(NADP) oxidoreductase-Aktivität, dadurch gekennzeichnet, daß man Mikroorganismen der Gattung Bacillus oder mit den entsprechenden Bacillusgenen transformierte Escherichia coli-Stämme in einem üb lichen Medium züchtet, aufschließt und das im Rohextrakt enthaltene Enzym nach chromatographischer und elektrophoretischer Aufreinigung mit die Polarität der wäßrigen Lösung herabsetzenden Substanzen versetzt oder mit Glucose oder Glucosederivaten kovalent verknüpft.

6. Verfahren nach Anspruch 5, dadurch gekennzeichnet, daß man das Enzym mit halogenierten Glucosederivaten in Gegenwart einer Lewis-Base umsetzt.

7. Verfahren nach den Ansprüchen 5 und 6, dadurch gekennzeichnet, daß man das Enzym mit 1-Brom-2,3,4,6-tetraacetylglucose in Gegenwart eines Silbersalzes umsetzt.

8. Enzym mit Creatinin:NAD(NADP) oxidoreductase-Aktivität.